# EUROPEAN PATENT SPECIFICATION

(11) **EP 1 233 784 B1**
(45) Date of publication and mention of the grant of the patent: **09.07.2008**
(21) Application number: 00992589.2
(22) Date of filing: 01.12.2000
(51) Int. Cl.: A61K 39/385, A61K 39/095, A61K 47/22, A61K 47/26

(54) **COMPOSITIONS AND METHODS FOR STABILIZING BIOLOGICAL MOLECULES UPON LYOPHILIZATION**
ZUSAMMENSETZUNGEN UND METHODEN ZUR STABILISIERUNG VON BIOLOGISCHEN MOLEKÜLEN NACH LYOPHILISIERUNG
COMPOSITIONS ET PROCEDES DE STABILISATION DE MOLECULES BIOLOGIQUES LORS DUNE OPERATION DE LYOPHILISATION

(30) Priority: 02.12.1999 US 168486 P
(43) Date of publication of application: 28.08.2002
(60) Divisional application: 07076078.0
(73) Proprietor: Novartis Vaccines and Diagnostics, Inc., Emeryville, CA 94608 (US)
(72) Inventor: ESIKOVA, Irina, Fremont, CA 94586 (US); WOLFE, Sidney, Lafayette, CA 94549 (US); THOMSON, James, Berkeley, CA 94706 (US); MANINDER, Hora, Danville, CA 94506 (US)
(74) Representative: Hallybone, Huw George
(86) International application number: PCT/US2000/042484
(87) International publication number: WO 2001/041800

(56) References cited:
- D.M. GRANOFF ET AL.: "MF59 adjuvant enhances antibody responses of infant baboons immunized with Haemophilus influenzae type b and Neisseria meningitidis group C oligosaccharide-CRM 197 conjugate vaccine." INFECTION AND IMMUNITY, vol. 65, no. 5, May 1997 (1997-05), pages 1710-1715, XP002172827 WASHINGTON, US
- L. BUSH ET AL.: "The formulation of recombinant factor IX: stability, robustness, and convenience." SEMINARS IN HEMATOLOGY, vol. 35, no. 2, suppl. 2, April 1998 (1998-04), pages 18-21, XP001010494 NEW YORK, US
- M.M. HO ET AL.: "Assessment of the stability and immunogenicity of meningococcal oligosaccharide C-CRM 197 conjugate vaccines." VACCINE, vol. 19, no. 7-8, 22 November 2000 (2000-11-22), pages 716-725, XP002172828 GUILDFORD, GB

## Description

### FIELD OF THE INVENTION

The present invention relates to compositions and methods for stabilizing biological molecules upon lyophilization, and methods for lyophilizing biological molecules.

### BACKGROUND OF THE INVENTION

Vaccines are widely used for the prevention and/or therapy of many different diseases. Meningococcal meningitis is one such disease for which prevention and/or therapy can be provided through a vaccine.

The meningococcal meningitis vaccine utilizes the meningococcal polysaccharide (PS) which is of relatively low immunogenicity. To overcome the relatively low immunogenicity of meningococcus polysaccharide, PS vaccines are conjugated to protein carriers to increase immunogenicity and provide long-term protection in young children. One such vaccine -- MenC-CRM 197 -- against meningococcus C (MenC) has been prepared using CRM 197 as the protein carrier (Costantino et al., Vaccine, 1992, Vol. 10, No. 10: 691-8).

Some problems are associated, however, with the use of the conjugate meningococcal polysaccharide vaccine MenC-CRM 197, and, indeed, with protein-containing vaccines in general. To extend the shelf-life of vaccines, formulations are frequently lyophilized. Lyophilization of MenC-CRM 197, however, can lead to protein aggregation during freezing and storage. In the case of MenC-CRM 197, the aggregates represent non-covalently bound, multimers of MenC-CRM 197 which apparently associate through hydrophobic interactions. The use ofpresent dissolution buffer formulations, *i.e.,* containing 10mM sodium phosphate (pH 7.2) as a buffer and 1.5% mannitol as an excipient, can yield aggregation as high as 9.5 to 10%. As aggregation increases, the concentration of available immunogen decreases. Therefore, a need exists for compositions and methods which overcome the problem of aggregation by stabilizing biological molecules against aggregation during lyophilization.
197 vaccine, is composed of two fragments, A and B, covalently bound to one another. Fragment A has been found to be stable and highly resistant to denaturation. Fragment B, however, is highly sensitive to denaturation and is subject to proteolytic breakdown during lyophilization. As proteolytic breakdown increases, the concentration of available functional immunogen decreases. Therefore, a further need exists for compositions and methods of preparation of biological molecules that will minimize breakdown during lyophilization and storage.

Although various theories exist that seek to explain and minimize aggregation and/or breakdown of biological molecules upon lyophilization, to date, no satisfactory techniques exist. Orii *et al.* report that glycerol and sugars protect biological materials from damage caused by freezing and thawing, presumably by minimizing pH changes (Orii et al., J. Biochem., 1977, 81:163-168)..

Arakawa *et al.* assessed the stabilization of proteins in aqueous solutions using various sugars (Arakawa et al., Biochemistry, 1982, 21: 6536-6544). Arakawa *et al.* link the protection of component proteins in aqueous solution to the cohesive force of sugars which are responsible for the increase in the surface tension of the water. This factor, however, is not relevant in lyophilized solutions. Arakawa *et al.,* do not address the stabilization of lyophilized solutions. Further, Arakawa *et al.* report that sucrose present in aqueous solutions actually decreases the activity of some enzymes.

Pikal *et al.* report that excipients must remain at least partially amorphous to be effective in stabilizing a protein, but cautioned that an amorphous excipient is not a sufficient condition for stability enhancement and, indeed, observed that the addition of amorphous excipients to protein solutions can actually destabilize the protein through molecular interactions between the excipient and protein (Pikal et al., Biopharm., 1990, 3, 26-29.) Pikal *et al.* state that high levels of buffers are to be avoided as this is likely to lead to crystallization of one of the buffer components and a subsequent large pH shift during freezing and that even buffers that remain amorphous are not suitable at high levels, as this normally lowers the collapse temperature and glass transition temperature of the dried material. The authors report that, in their experience, a low level of buffer tends to remain amorphous yet does not dramatically affect the collapse temperature or glass transition temperature. Pikal *et al.,* however, does not define high or low levels of buffer. In a later report *al*., however, Pikal *et al.* report that, based on reverse-phase HPLC, aggregation was at a maximum in 1.69 mM phosphate buffer while decomposition was at a minimum (Pikal et al., Pharm. Res., 1991, 8:427-436). The authors concluded, thus, that the level of phosphate buffer does not appear to be a relevant factor.

Izutsu *et al.* report that maintaining mannitol in an amorphous state stabilizes β-galactosidase (Izutsu et al., Pharm. Res., 1993, Vol. 10, No. 8, 1232-1237). Izutsu *et al.* report that in 200 mM sodium phosphate buffer, mannitol, at 200-500mM is amorphous and acted to stabilize β-galactosidase. In 10 and 50 mM sodium phosphate buffer, mannitol at 50-100nM is amorphous and exhibited a stabilizing effect. Izutsu *et al.* also report that glucose in sodium phosphate buffer protects enzyme activity, but does not provide any data on concentrations of glucose suitable to attain such an effect.

Bush et al. discloses a stable lyophilized recombinant factor IX formulation containing 10mmol/L histidine, 0.26 mol/L glycine, 1 % sucrose and 0.005 % polysorbate 80. (Bush et al., Seminars is Hematology, 1998, Vol. 35, No 2, suppl. 2, 18-21).

The protective effect of select amino acids on lyophilized enzyme solutions has also been tested (Seguro et al., Cryobiology, 1990, 27: 70-79). The protective effects of monosodium glutamate (MSG) and lysine hydrochloride (Lys-HCl) during lyophilization using lactate dehydrogenase in solution were assessed. Seguro *et al.* note that the addition of either MSG or Lys-HCl to the enzyme solution reduces the loss of enzyme activity from about 40% (control using deionized water) to 20-25% (using MSG or Lys-HCl). The authors suggest that neither the salt concentration nor the ionic strength is important in the stabilization of the dissolved enzyme. Seguro *et al.* report that the most relevant factor in freeze denaturation is either the termination of supercooling phenomena produced by the additives, or the phase change from water to ice.

Procedures for lyophilization of various compositions have been described in several references (DeLuca, J. Vac. Sci. Technol., 1977, Vol. 14, No. 1, 620-629; DeLuca et al., J. Pharm. Sci., 1965, Vol. 54, No. 4, 617-624; Pikal et al., Biopharm., 1990, 3, 26-29). The methods for lyophilizing described in these references include a series of cycles.

DeLuca *et al.* (1965) report that the eutectic temperature of a product is an important factor in the design of lyophilization cycles and that the eutectic temperatures for various organic and inorganic salts are similar (Table I). The authors state that other factors important in the design of lyophilization cycles include the supercooling effect and the thermal conductive property of the frozen mass. DeLuca *et al.* (1965) report that neither mannitol nor sucrose exert a significant effect on the solubility of organic salts or the eutectic temperature of the system. DeLuca *et al.* (1977) report that the eutectic point of the additive should not be lower than the drug as this will affect cycle times and suggests adding mono-and di-basic phosphate and sodium chloride to improve the characteristics of the finished cake. The authors conclude that it may be possible to optimize cycle time by determining the eutectic temperatures and the supercooling properties of the product.

A need exists for compositions and methods for stabilizing biological molecules upon lyophilization.

### SUMMARY OF THE INVENTION

The present invention arises from the surprising discovery that it is possible to stabilize biological molecules against aggregation and breakdown during and following, *i. e.* "upon", lyophilization, using a dissolution buffer comprising an amorphous excipient and an amorphous buffer.

According to one aspect, the present invention provides a dissolution buffer comprising at least one amorphous excipient, at least one amorphous buffer, and at least one MenC immunogen.

According to another aspect, the present invention provides a method for stabilizing one or more MenC immuogens upon lyophilization. The method comprises dissolving the MenC immuogen in a dissolution buffer comprising at least one amorphous excipient and an amorphous buffer to form a mixture, and then lyophilizing the mixture.

In yet another aspect, the present invention provides a method for storing one or more MenC immunogens. The method comprises dissolving the MenC immunogen in a dissolution buffer comprising at least one amorphous excipient and an amorphous buffer to form a mixture, and then lyophilizing the mixture.

In still another aspect, the present invention provides a method for lyophilization of a MenC immunogen. The method comprises dissolving a MenC immunogen in a dissolution buffer comprising at least one amorphous excipient and an amorphous buffer to form a mixture,
loading the mixture from about 10°C to about 20°C under from about 1000 Pa to about 2000 Pa of pressure, freezing the mixture from about 1 hour to about 4 hours to a temperature from about -70°C to about -50°C under from about 1000 Pa to about 2000 Pa of pressure, drying the mixture for from about 18 hours to about 30 hours at a temperature from about -50°C to about -35°C under a pressure from about 5 Pa to about 10 Pa, and drying the mixture for from 2 hours to about 12 hours at a temperature from about 10°C to about 20°C under a pressure from about 5 Pa to about 10 Pa.

In another aspect, the present invention provides a method for lyophilization of a MenC immunogen. The method comprises
dissolving a MenC immunogen in a dissolution buffer comprising at least one amorphous excipient and an amorphous buffer to form a mixture, loading the mixture at about 10°C under 1000 Pa of pressure, freezing the mixture for about 2 hours to a temperature of about -50°C under about 1000 Pa of pressure, drying the mixture for about 24 hours at a temperature of about -35°C under a pressure of about 5 Pa, and drying the mixture for about 8 hours at a temperature of about 20°C under a pressure of about 5 Pa.

In another aspect, the present invention provides a method for stabilizing a MenC vaccine upon lyophilization. The method comprises dissolving the MenC vaccine in a dissolution buffer comprising at least one amorphous excipient and an amorphous buffer to form a mixture, and lyophilizing the mixture.

### BRIEF DESCRIPTION OF THE DRAWINGS

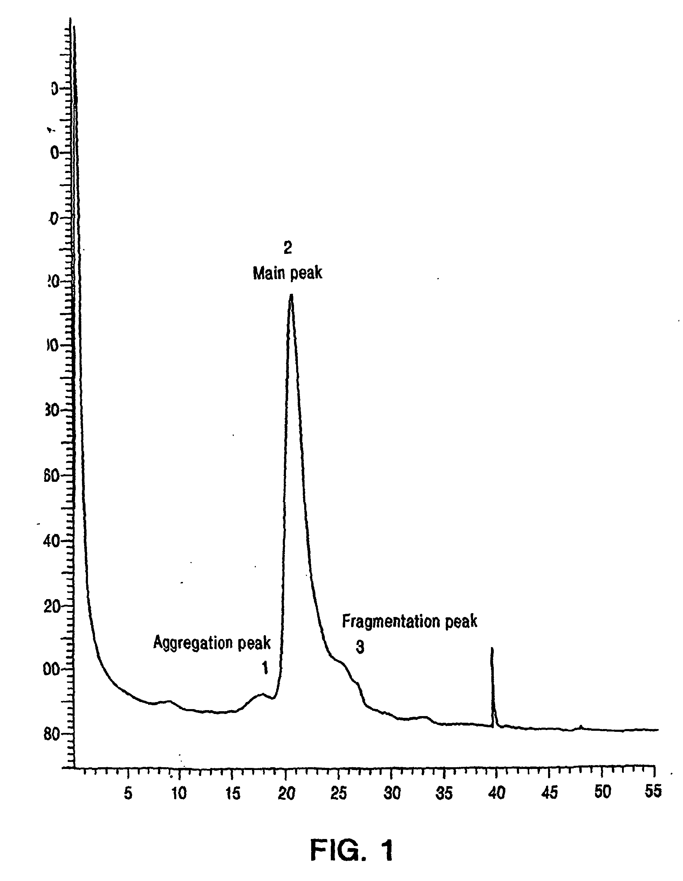

Figure 1 depicts a chromatogram of MenC-CRM 197 obtained by Size Exclusion Chromatography (SEC). The main peak is seen between 19 and 24 minutes. A peak representing aggregation products is seen at about 15-17 minutes, and a peak representing fragmentation products is seen at greater than 24 minutes.

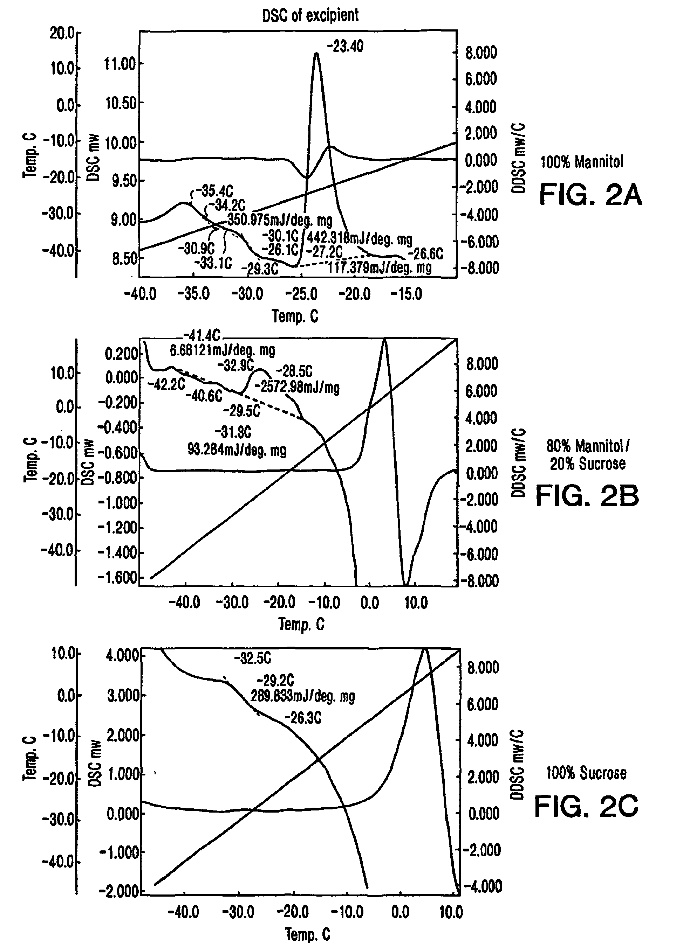

Figures 2A-C depict Differential Scanning Calorimetry (DSC) diagrams of a mannitol buffer (A), a mannitol-sucrose buffer (B), and a sucrose buffer (C). Replacement of 25% of the mannitol with sucrose in the buffer resulted in a decrease of the re-crystallization peak and a shift of the glass transition temperatures.

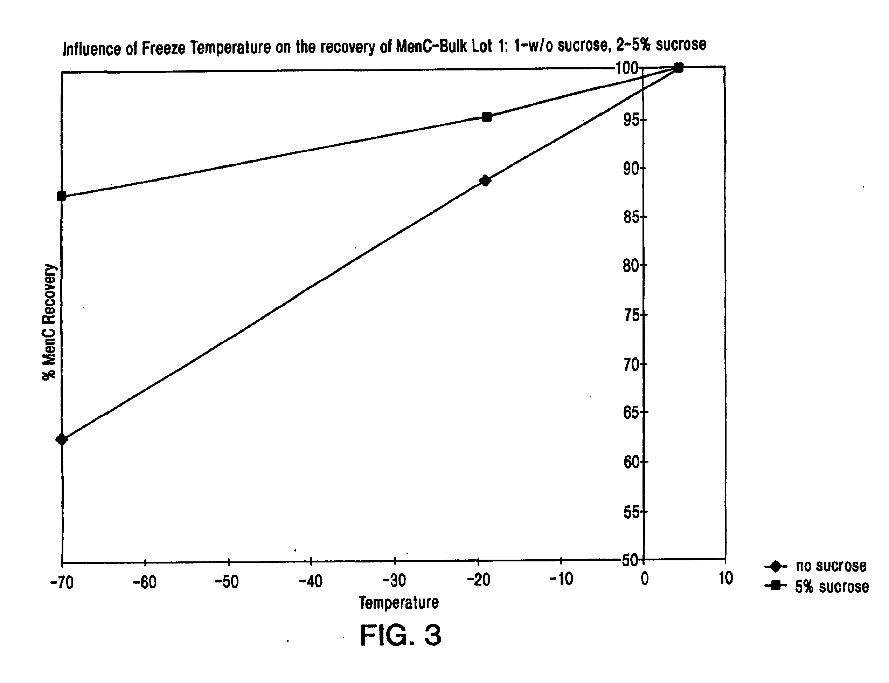

Figure 3 depicts the effects of freeze temperature and presence of sucrose on the stability of MenC-CRM 197 formulation upon lyophilization as determined by percent recovery of MenC. MenC-CRM 197 exhibits greater stability in the presence of 5.0% sucrose when compared to formulations without sucrose.

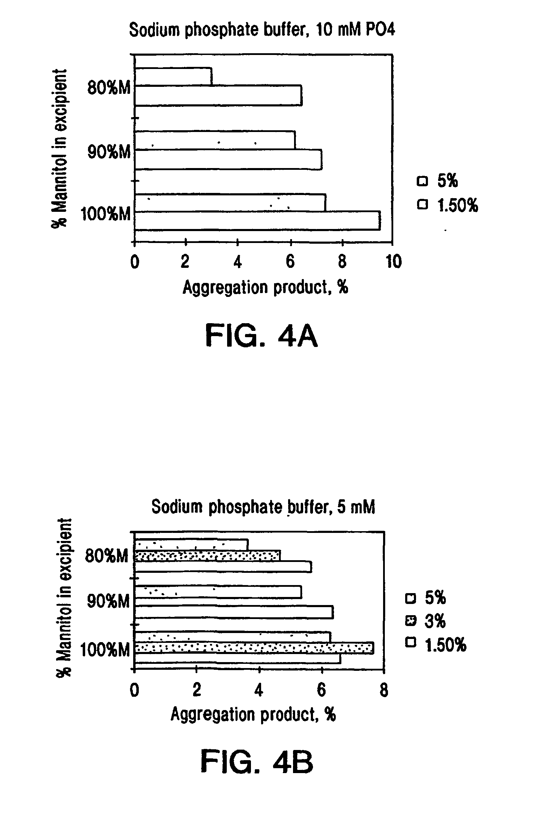

Figures 4A-B show the effect of the excipient-buffer composition on the aggregation of MenC-CRM 197 as determined by Size Exclusion Chromatography. Figure 4A depicts the use of a 10mM sodium phosphate buffer containing varying amounts of mannitol and/or sucrose. Figure 4B depicts the use of a 5mM sodium phosphate buffer containing varying amounts of mannitol and/or sucrose. As the amount of amorphous excipient, in this case sucrose, in the formulation increased, aggregation of MenC-CRM 197 decreased.

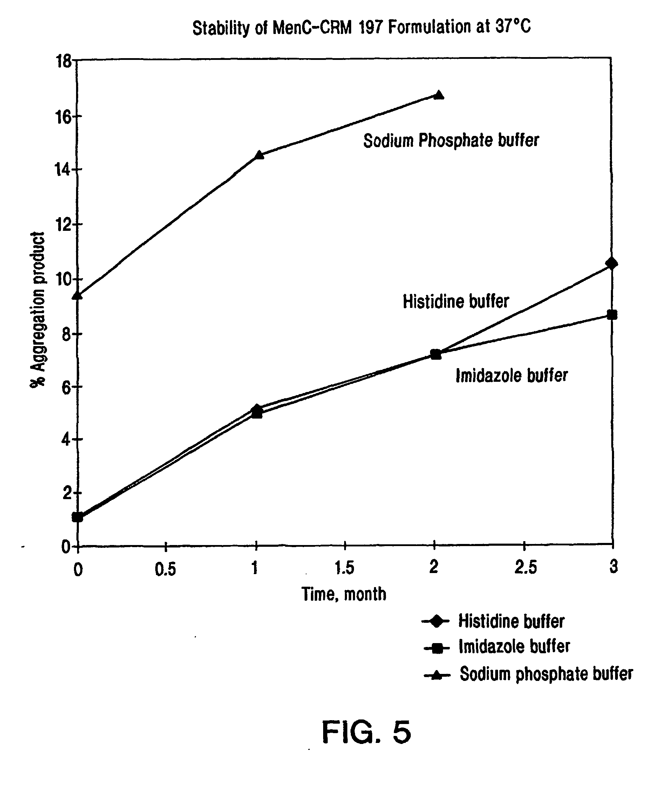

Figure 5 depicts the effect of stress (37°C) on the aggregation of MenC-CRM 197 as determined by Size Exclusion Chromatography. Formulations with amorphous buffers (either histidine or imidazole) demonstrated less aggregation after prolonged incubation at 37°C than did formulations with non-amorphous buffers (sodium phosphate buffer).

### DETAILED DESCRIPTION OF THE INVENTION

The present invention provides buffer compositions, biological molecule compositions, and methods for the preparation and stabilization of biological molecules by reducing aggregation and breakdown of biological molecules upon lyophilization. The compositions and methods involve replacing the crystallizable (non-amorphous) buffers and excipients with completely or partially amorphous buffers and excipients.

As used herein, the phrase "dissolution buffer" refers to the solution in which the protein or proteins are dissolved for lyophilization. The "dissolution buffer" comprises at least one amorphous excipient and at least one amorphous buffer. In a preferred embodiment, the dissolution buffer further comprises a MenC immunogen. In a more preferred embodiment, the dissolution buffer further comprises MenC-CRM 197.

As used herein, the term "biological molecule" includes, but is not limited to, proteins, nucleic acids, and saccharides. Biological molecules may be used individually or in combination with other biological molecules. In a preferred embodiment of the present invention, the biological molecule is an immunogen. In a more preferred embodiment, the biological molecule is a MenC immunogen. In an even more preferred embodiment, the biological molecule is MenC-CRM 197.

As used herein, the term "protein" refers to polypeptides, peptides, and analogs thereof "Protein" also includes polypeptides and peptides which are conjugated to other biological molecules. In a preferred embodiment of the present invention, the protein is an immunogen.

As used herein, the term "immunogen" refers to any compound capable of eliciting a cellular and/or humoral immune response when in contact with a cell, and includes, without limitation, vaccines and compositions comprising immunogens. Preferably, an antibody response and a T cell response are elicited. Such immunogens would be expected to be useful in prophylactic and therapeutic applications as well as for research applications including the generation of antibodies.

As used herein, the term "nucleic acid" refers to oligonucleotides and polynucleotides, and includes DNA and RNA.

As used herein, the term "saccharides" includes oligosaccharides and polysaccharides.

As used herein, the phrase "amorphous excipient" refers to an excipient which remains amorphous upon lyophilization. Excipients which remain amorphous under certain conditions but may crystallize under other conditions are included. Conditions which maintain the amorphous nature of the excipient are included in the present invention. Amorphous excipients are well known to those skilled in the art and include, without limitation, sugars, including sucrose, lactose, and, under conditions described herein, mannitol. Amorphous excipients also may include, under conditions well known to those of skill in the art, without limitation, celluloses, especially sorbitol. The amorphous excipients can be used either individually or as a mixture containing two or more different amorphous excipients. In a preferred embodiment, the amorphous excipient is sucrose. In a more preferred embodiment, the final sucrose concentration in the dissolution buffer is from about 1.5% to about 5%.

As used herein, the phrase "MenC vaccine" refers to a vaccine comprises at least one MenC immunogen. In a preferred embodiment, the MenC vaccine comprises MenC-CRM 197.

As used herein, the phrase "amorphous buffers" refers to buffers which remain amorphous upon lyophilization, e.g., organic buffers. Buffers which remain amorphous under certain conditions but may crystallize under other conditions are included. Conditions for maintaining the amorphous nature of the buffer are also included. Amorphous buffers are well known to those skilled in the art and include, without limitation, imidazole buffer and histidine buffer, piperidine, and diisopropylethylamine. In a preferred embodiment, the amorphous buffer is imidazole buffer. In a further preferred embodiment, the amorphous buffer is imidazole buffer and has a concentration of about 10mM.

As used herein, the term "stabilizing" is used synonymously with inhibiting, reducing, suppressing, decreasing, diminishing, and lowering aggregation and/or breakdown. The present invention includes methods that substantially inhibit aggregation and/or breakdown of biological molecules. Substantial inhibition of aggregation or breakdown refers to a reduction of aggregation or breakdown from about 25% to about 100% compared to controls which use crystallizable buffers and/or excipients. Preferably, aggregation or breakdown is inhibited by about 50%, more preferably by about 75%, and even more preferably by about 100%.

As used herein, the term "upon lyophilization" refers to the period during lyophilization of the biological molecules and during subsequent storage of the lyophilized biological molecules.

As used in this specification and the appended claims, the singular forms "a", "an", and "the" include plural references unless the content clearly dictates otherwise. Thus, for example, reference to "an immunogen" includes a mixture of two or more such immunogens.

In accordance with the present invention, methods for lyophilization of biological molecules are also provided. The methods comprise dissolving the biological molecule in a dissolution buffer which comprises at least one amorphous excipient and an amorphous buffer to form a mixture, and then lyophilizing the mixture. In one embodiment of the invention, lyophilization includes loading the samples at 20°C, freezing the samples to about -50°C for about 2 hours, and drying at about -35°C for about 24 hours under about 25 microns of vacuum pressure and at a ramp rate of about 25°C per hour. In a preferred embodiment, the biological molecule is a MenC immunogen. In a more preferred embodiment, the biological molecule is MenC-CRM 197. Through the practice of the present invention, the biological molecule is stabilized against aggregation and breakdown.

This invention satisfies a long felt-need for compositions and methods for preparing biological molecules which reduce aggregation and minimize breakdown upon lyophilization. By replacing non-amorphous excipients and non-amorphous buffers in the dissolution buffer with amorphous excipients and amorphous buffers, a reduction of aggregation and breakdown during lyophilization and subsequent storage is achieved.

### Materials and Methods

The following materials and methods were used unless otherwise noted.

The group C menningococcal polysaccharide (MenC) is a 2-9 linked homopolymer of sialic acid (N-acetyl neuraminic acid) partially O-acetylated in position C7 and/or C8. CRM 197 is a mutant of the wild-type diphtheria toxin (Constantino et al., Vaccine, 1992, Vol. 10, No. 10, 691-8). Replacement of the glycine present at amino acid position 52 with glutamic acid leads to complete loss of enzymatic activity. To prepare the conjugate, the capsular polysaccharide of *Neisseria meningitidis* group C was hydrolyzed and the resulting oligosaccharide (OS) was coupled to CRM 197. The coupling of CRM 197 to the capsular polysaccharide was based on selective end-reducing group activation of the oligosaccharide and subsequent coupling to the protein through a special spacer (Constantino et al, Vaccine, 1992, 10: 691-698). The ratio of sialic acid to CRM 197 in the conjugate is about 0.6. The average length of oligosaccharides was estimated by ion exchange chromatography (Mono-Q analysis, elution on Q Sepharose FF™) as 14.2. The K_{d} of the oligosaccharide was between 0.33 and 0.41, and the free saccharide concentration was about 2.5%.

MenC-CRM 197 Bulk Lot 1 ("MenC Bulk") consists of about 1.045 mg/mL CRM 197, and about 0.636 mg/mL MenC antigen (as measured by sialic acid content), in 10mM sodium phosphate buffer (pH=7.2) and is stored at 4°C. All MenC-CRM 197 solutions disclosed herein were filtered through 0.22 µm sterile Millipore™ filters before testing. The bulk solution is diluted before lyophilization.

MenC conjugate vaccine lyophilized antigen (MenC-CRM 197) obtained from Siena had the following composition: 12 µg (as measured by sialic acid content) of meningococcal C oligosaccharide antigen (Siena, Italy) conjugated to 19.7 µg of CRM 197, 9 mg of mannitol, and 10mM phosphate buffer, pH 7.2. Prior to administration, the lyophilized antigen is solubilized with 0.6 mL of aluminum hydroxide adjuvant. Of this solution, 0.5 mL is injected.

DSC (Differential Scanning Calorimetry): The SII Seiko instrument with a Hewlett-Packard operating system was used to calculate DSC by analyzing additive crystallinity. Sample solutions of the excipient solutions were frozen at a ramp rate of 10°C/minute to a temperature of about -50°C. Using dry nitrogen as the atmosphere, thermal changes were monitored while warming samples at various ramp rates (0.625°C/minute to 10°C/minute). The effect of excipient composition on thermal change was assessed.

Dialysis of MenC Bulk was performed using Spectra/Por Membrane^{®} (Spectrum Laboratories, Laguna Hills, CA) with a 10,000 MW cutoff to replace the sodium phosphate buffer with the various buffers tested herein. Following dialysis, the MenC Bulk solution was diluted with various volumes of buffer and appropriate amounts of excipient to yield a desired protein concentration of 1.045 mg/mL. Sodium phosphate, histidine hydrochloride, and imidazole hydrochloride buffers, all pH 7.2, were used in the formulations. Buffer concentrations varied from 5 to 10 mM. Excipients used were mannitol, mannitol-sucrose mixtures, or sucrose. Excipient concentrations were 1.5%, 3%, or 5%. In some formulations, Tween-80^{®} was also added. After preparation, all formulated MenC Bulk solutions were filtered through 0.22 µm sterile Millipore filters and stored at 4°C.

Lyophilization: The final product was prepared by lyophilization of the MenC Bulk. The Freeze-Drying Cycle included several different stages and was different for mannitol (A*), mannitol-sucrose (B), and sucrose (C) formulations (see Table 1).

HPSEC (High Performance Size Exclusion Chromatography) analysis: TSK GEL^{R} G4000SW_{XL} columns (30cm x 7.8 mm inner diameter) (TosoHaas, Montgomeryville, PA), columns of small-particle hydrophilic gels for SEC (Size Exclusion Chromatography) separation in aqueous mobile phase were used for analysis of MenC-CRM 197 vaccine following lyophilization and solubilization. According to the instructions supplied by the manufacturer, the number of theoretical plates was greater than 16,000 and the asymmetry factor was from 0.7 to 1.6.

HPSEC analysis showed the retention times of 18.3 minutes and 22.8 minutes for MenC-CRM 197 (Siena) and MenC Bulk, respectively. The recovery of MenC as measured with the fluorescence detector was about 95% (value calculated from 5 measurements for 10 µL injection volumes). Increasing injection volume led to aproportional increase in the area of major peaks. The reproducibility of injections was high with a standard error (standard deviation x 100/Average peak area) less than 1%. The accuracy of 10 injections (each 20 µL) from different vials was also high (average value=19.83 µL, standard deviation = 0.085 µL). The minimum injection volume (standard deviation = 0.1 µL from 20 µL) was found to be 35 µL.

A typical chromatogram of MenC-CRM 197 (lyophilized) is presented in Figure 1, and shows a broad, non-symmetrical peak between 19-24 minutes (main peak). After sample stress, two additional peaks appeared; one related to the product with a higher molecular weight (an aggregation product) with a retention time between 15-17 minutes, and another with a lower molecular weight (fragmentation product) with a retention time over 24 minutes.

For the column chromatography the flow rate was 0.5 mL/min., pressure was between 350-400 psi., the mobile phase was 100 mM phosphate buffer (pH= 7.2) with 200 mM (NH₄)₂SO₄ (HPLC grade, Bio-Rad), at room temperature. Sample emissions were detected with the UV detector at 214 nm and fluorescence detector at 280 nm Ex. and 340 nm Em. A gel filtration standard containing Gamma-globulin, ovalbumin, myoglobin, and vitamin B-12 (Bio-Rad) was used as a standard. Analysis was performed using a two-pump Waters 510 HPLC system consisting of a Waters Automated Gradient Controller and Model 510 pumps, a Waters 486 Tuneable Absorbance Detector, a Waters 712 WISP injection system, and a Hitachi F1 Detector L-7480 with a Xe lamp.

The following examples are meant to illustrate the invention and are not to be construed to limit the invention in any way. Those skilled in the art will recognize modifications that are within the spirit and scope of the invention.

### EXAMPLES

### Example 1: Freeze Experiment with pH indicator

Eighteen different formulations were prepared to study the influence of the solution composition on the pH change during freezing. Table 2 shows the variations of phosphate buffer concentrations, excipient concentrations, and mannitol-sucrose ratio in these formulations. The initial pH value for all formulations was pH 7.2. The freeze experiment was performed in a lyophilizer chamber and included several steps. Samples were loaded into the lyophilizer chamber and held for 0.2 hours at 20°C. Samples were then cooled to -50°C with a ramp rate of 60°C/minute and then held at -50°C for 0.5 hours. Next, samples were warmed to -20°C with a ramp rate of 60°C/minute and held at -20°C for 0.5 hours. Samples were cooled to -50°C with a ramp rate of 60°C/minute and then warmed to -20°C with a ramp rate of 60°C/minute.

Following freeze-drying, pH measurements were taken at various time points and temperatures. The experiment was performed with a universal pH indicator that changed color with pH change. Each color was related to the relevant pH. pH measurements were taken at time zero at 20°C (point 1), after 3 hours at -20°C (point 2), after 4 hours at -50°C (point 3), and after 5 hours at -20°C (point 4). Samples were held at -20°C overnight and the next measurements were taken at -20°C after 34.5 hours (point 5). A statistical study of 144 vials containing various formulations (8 vials for each formulation; 18 formulations) was performed at -20°C after 34.5 hours.

It is known that phosphate buffers change pH during freezing from a temperature of 20°C (2) to -20°C (4). The formulations containing either a mannitol or mannitol-sucrose mixture displayed fewer changes in pH (see Table 2). At -50°C, pH was between about 5 and 5.5, and at -20°C, pH was between about 6 to 6.5. No significant changes in pH of the various formulations were observed during the initial 5 hours. However, certain color changes took place after 34.5 hours, namely the appearance of orange and red spots at the interface of the yellow and orange solid. The area of the red spots increased with time. This observation demonstrated that the crystallization of phosphate salts was a slow and random process. Further, two salts, monosodium phosphate and disodium phosphate, had different crystallization points, -9.7°C and -0.5°C, respectively.

Statistical study of the pH changes during freezing indicated that the observations could be divided into 3 groups:

| | |
|---|---|
| Group 1 | pH 6.0 |
| Yellow | pH 6.0 and spots with pH 5.5 |
| | |
| Group 2 | pH 5.5 |
| Orange | pH 5.5 and greater than 5 spots with pH 5.0 |
| | |
| Group 3 | pH 5.0 |
| Red | pH 5.0- 5.5 and greater than 5 spots with pH 5.0 |

The percentage of the samples belonging to each group was calculated using the formula: % = n x 100/N, where n was the number of samples in each group and N was the total number of samples analyzed (in this case N=8) (see Table 2).

The statistical analysis presented in Table 2 showed that, to a large extent, the formulations containing mannitol belonged to Groups 2 and 3 while the formulations with a high sucrose content belonged to Group 1. Thus, replacement of a portion of the mannitol with sucrose resulted in fewer pH changes. The more sucrose present in the formulation resulted in a smaller pH change. The most notable results were obtained for either 1.5% excipients, (with either 80%, 90%, or 100% mannitol) in 10mM phosphate buffer, and 5% excipients (with 80% or 90% mannitol) containing sucrose in 5mM phosphate buffer, each of which lead to 100% of the samples falling into the yellow group.

Addition of inert organic compounds such as mannitol and sucrose decreased the pH changes of the phosphate buffer. Such an effect of these additives might be related to the increased viscosity of the formulation which might inhibit crystal growth and precipitation of the phosphate salts. Because visco-elastic properties of the solution could affect the temperature of crystallization (5), the properties prevented crystallization of the salt upon cooling giving a supercooled liquid dispersed within an ice crystal meshwork.

### Example 2: Effect of Buffer Selection on Recrystallization

It has been reported that mannitol in water shows an exothermic peak at about -25°C due to the crystallization of mannitol (Gatlin et al., J. Parent. Drug. Assoc., 1980, 344:398-408). In the presence of sodium phosphate buffer, the exothermic peak temperature differed from that in aqueous solution (Williams et al., J. Parent Sci. Tech., 1991, 45:94-100; Seguro et al., Cryobiology, 1990, 27: 70-79). The thermal behavior differences observed might be related to the changes in crystallinity (Izutsu et al., Pharm. Res., 1993, 10, No. 8, 1232-1237).

The DSC diagram in Figures 2A-C show that a 3% mannitol solution in 10mM sodium phosphate buffer, pH 7.2, caused exothermic re-crystallization at -23.5°C (ΔH= - 7735.2 mJ/mg) and endothermic glass transition at -32°C (ΔCp=351 mJ/deg.mg) and -30.1°C (ΔCp=442mJ/deg.mg) (Figures 2a and 2b). Replacement of 25% of the mannitol with sucrose resulted in a decrease of the re-crystallization peak (ΔH= -2572 mJ/mg) and a shift of the glass transition temperatures to -41.4°C (ΔCp=6.7mJ/deg.mg) and -31.0°C (ΔCp=93mJ/deg.mg). Thermal analysis of the sucrose solution (Figure 2C) in the sodium phosphate buffer showed only the glass transition at -29.2°C (ΔCp=289.8mJ/deg.mg). Thus, the addition of sucrose to the crystallizable mannitol solution resulted in the formation of amorphous mannitol during cooling and prevented crystallization.

### Example 3: Stabilization of MenC Bulk with sucrose

Stabilization of MenC Bulk with sucrose was shown in a freeze experiment (Fig. 3). Fourteen formulations of MenC Bulk were prepared as described above with various amorphous excipient compositions and phosphate buffer concentrations (Table 3). The formulations were lyophilized according to method B (see Table 1). The lyophilization included freezing with annealing as previously developed by Siena, Italy (Carpenter *et al.,* **1988**, 25: 244-255). The suppression of aggregation during lyophilization as determined by Size Exclusion Chromatography was used to gauge stabilization. As shown in Figure 4, suppression of aggregation was found to be directly related to the sucrose content.

### Example 4: Replacement of the sodium phosphate buffer with amorphous buffer increases the stability of the MenC Bulk formulations.

Histidine-histidine hydrochloride and imidazole-imidazole hydrochloride solutions, both pH 7.2, were used as buffers. Pure sucrose without mannitol was used to stabilize the protein. Various formulations were tested in an attempt to stabilize MenC Bulk. These formulations were lyophilized according to method C (see Table 1).

The freeze drying cycle included a single freeze without annealing and primary drying at -35°C, a temperature lower than the glass transition temperature of sucrose. Because of the amorphous state of the excipient during lyophilization, the total freeze cycle time was increased as was the vacuum pressure. The results presented in Table 3 demonstrate that aggregation during lyophilization was completely prevented. In control experiments, i.e., non-amorphous buffers and/or excipients, aggregation was higher than that observed for test solutions. For example, aggregation was 3.3% in 10mM sodium phosphate buffer with sucrose and 9.5% in 10mM sodium phosphate buffer with mannitol. Aggregation was 0% in 10mM imidazole buffer with 1.5% sucrose and 0.78% in 10mM histidine buffer with 1.5% sucrose.

### Example 5: Stability study

MenC-CRM 197 solid samples were stressed after lyophilization at 4°C. 25°C, or 37°C in an incubator for a period of 3 months. At selected time points, samples were removed from the incubator, reconstituted with 0.6 mL distilled water, and analyzed by HPSEC. All formulations were stable at 4°C, i.e., did not show an increase in aggregation upon storage.

Samples of MenC-CRM 197 (Sienna) showed higher aggregation during stress testing than did formulations containing either imidazole or histidine buffers (Fig. 5). Use of the histidine or imidazole buffers in the MenC-CRM 197 formulation increased the vaccine stability. Although the inventors do not wish to be bound to this mechanism, it is thought that stabilization maybe due to the interaction of the tryptophan of CRM 197 with the structurally similar imidazole ring.

**Table 1 Lyophilization process for MenC CRM 197 Formulations**

| Process step | | Total process time hr | Shelf Temperature °C | Pressure Pascals (Pa) |
|---|---|---|---|---|
| Loading | A* | 0.0 | 20 | 1000 |
| | B | 0.0 | 20 | 1000 |
| | C | 0.2 | 10 | 1000 |
| Freezing I | A* | 2.0 | -55 | 1000 |
| | B | 1.0 | -50 | 1000 |
| | C | 2.0 | -50 | 1000 |
| Annealing | A* | 2.0 | -24 | 1000 |
| | B | 2.0 | -25 | 1000 |
| Freezing II | A* | 3 | -37 | 1000 |
| | B | 1.0 | -50 | 1000 |
| Primary drying | A* | 15 | 37 | 21 |
| | | 1.0 | -37 | 1.5 |
| | B | 24.0 | -20 | 10 |
| | C | 24.0 | -35 | 5 |
| Secondary drying | A* | 2.0 | 30 | 1.5 |
| | B | 8.0 | 20 | 10 |
| | C | 8.0 | 20 | 5 |

| | | | | |
|---|---|---|---|---|
| A* -previously developed by Biocine Freezing ramp -60°C/h, drying ramp - 25°C/h Special steps are pre-aeration (N₂/air, pressure 0.85 bar), stoppering, aeration (N₂/air), and storage at 4°C. A= Freeze-Drying Cycle for mannitol B= Freeze-Drying Cycle for mannitol-sucrose C= Freeze-Drying Cycle for sucrose | | | | |

**Table 2 The pH changes during the freeze experiment with pH indicator**

| Formulation | | | | pH | | | | Yellow group samples, % |
|---|---|---|---|---|---|---|---|---|
| % Mannitol | % Excipient | mM PO₄ | 0 | 3 h | 4h | 5h | 34.5h | |
| | | | 20°C | -20 | -50 | -20 | -20 | |
| 100 | 1.5 | 10 | 7 | 6 | 5 | 6 | 6(5.5)* | 100 |
| 100 | 1.5 | 5 | 7 | 6 | 5.5 | 6-5.5 | 6(5.5)* | 78 |
| 90 | 1.5 | 10 | 7 | 6 | 5.5 | 6.5-6 | 6(5.5)* | 100 |
| 90 | 1.5 | 5 | 7 | 6 | 5 | 6 | 6.5-5(6)* | 100 |
| 80 | 1.5 | 10 | 7 | 6 | 5 | 6 | 6(5.5)* | 100 |
| 80 | 1.5 | 5 | 7 | 6 | 5 | 6.5 | 6 | 100 |
| | | | | | | | | |
| 100 | 3 | 10 | 7 | 6(5.5)* | 5 | 6-5.5 | 6 | 50 |
| 100 | 3 | 5 | 7 | 6(5.5)* | 5.5 | 6 | 6 | 63 |
| 90 | 3 | 10 | 7 | 6 | 6-5.5 | 6-5.5 | 7(5)* | 25 |
| 90 | 3 | 6 | 7 | 6 | 5 | <6 | 6(5.5-5)* | 87 |
| 80 | 3 | 10 | 7 | 6 | 5 | 6 | 6 | 75 |
| 80 | 3 | 5 | 7 | 6 | 5.5-6 | 6 | 6.5-6(6)* | 90 |
| | | | | | | | | |
| 100 | 5 | 10 | 7 | 6(5.5)* | 5.5 | 6-5.5 | 6 | 77 |
| 100 | 5 | 5 | 7 | 6 | 5.5 | 6.5 | ? | 25 |
| 90 | 5 | 10 | 7 | 6 | 5 | 6.5 | 6(5.5) | 37 |
| 90 | 5 | 5 | 7 | 6 | 5 | 6.5-6 | 6.5(5.5)* | 100 |
| 80 | 5 | 10 | 7 | 6(5.5)* | 5.5 | 6.5 | 6.5-6 | 88 |
| 80 | 5 | 5 | 7 | 6 | 5.5 | 6 | 6 | 100 |

| | | | | | | | | |
|---|---|---|---|---|---|---|---|---|
| * The pH value for spots on the surface of solid sample | | | | | | | | |

**Table 3 Aggregation of Imidazole and Histidine formulation of MenC-CRM 197 vaccine under lyophilization**

| # | Formulation | % Aggregation product |
|---|---|---|
| 1 | 10 mM Imidazole, 1.5% sucrose | 0 |
| 1a | 10 mM Imidazole, 1.5% sucrose, 0.05% Tween 80 | 0 |
| | | |
| 2 | 10 mM Imidazole, 3% sucrose | 0.21 |
| 2a | 10 mM Imidazole, 3% sucrose, 0.05% Tween 80 | 0 |
| | | |
| 3 | 10 mM Imidazole, 5% sucrose | 0 |
| 3a | 10 mM Imidazole, 5% sucrose, 0.05% Tween 80 | 0 |
| | | |
| 4a | 10 mM Histidine, 1.5% sucrose, 0.05% Tween 80 | 0 |
| 4 | 10 mM Histidine, 1.5% sucrose | 0 |
| | | |
| 5 | 10mM Histidine 3% sucrose | 0.78 |
| 5a | 10 mM Histidine, 3% sucrose, 0.05% Tween 80 | 0 |
| | | |
| 6 | 10 mM Histidine, 5% sucrose | 0 |
| 6a | 10 mM Histidine, 5% sucrose, 0.05% Tween 80 | 0 |
| Control: | 10 mM NaPO₄, pH 7.2, 1.5% sucrose | 3.3 |
| Siena: | 10 mM NaPO₄. pH 7.2.1.5% mannitol | 9.5% |

## Claims

1. A dissolution buffer comprising at least one amorphous excipient, at least one amorphous buffer, and at least one meningococcus C (MenC) immunogen.

2. The dissolution buffer of claim 1 wherein the amorphous buffer is an organic buffer.

3. The dissolution buffer of claim 2 wherein the organic buffer is selected from the group consisting of imidazole buffer and histidine buffer.

4. The dissolution buffer of claim 1 wherein the amorphous excipient is a sugar.

5. The dissolution buffer of claim 4 wherein the sugar is sucrose.

6. The dissolution buffer of any preceding claim wherein the meningococcus C (MenC) immunogen is MenC-CRM 197.

7. A method for stabilizing one or more meningococcus C (MenC) immunogens upon lyophilization comprising;
(a) dissolving the meningococcus C (MenC) immunogen in a dissolution buffer comprising at least one amorphous excipient and an amorphous buffer to form a mixture, and
(b) lyophilizing the mixture.

8. The method of claim 7 wherein the meningococcus C (MenC) immunogen is MenC-CRM 197.

9. A lyophilized composition stabilized according to the method of claim 7 comprising at least one amorphous excipient, at least one amorphous buffer, and at least one meningococcus C (menC) immunogen.

10. A method for storing one or more meningococcus C (MenC) immunogens comprising;
(a) dissolving the meningococcus C (MenC) immunogen in a dissolution buffer comprising at least one amorphous excipient and an amorphous buffer to form a mixture, and
(b) lyophilizing the mixture.

11. The method of claim 10 wherein the meningococcus C (Men C) immunogen is MenC-CRM 197.

12. A method for lyophilization of a meningococcus C (Men C) immunogen comprising;
dissolving a meningococcus C (Men C) immunogen in a dissolution buffer comprising at least one amorphous excipient and an amorphous buffer to form a mixture;
loading the mixture from 10°C to 20°C under from 1000 Pa to 2000 Pa of pressure;
freezing the mixture from 1 hour to 4 hours to a temperature from -70°C to -50°C under from 1000 Pa to 2000 Pa of pressure;
drying the mixture for from 18 hours to 30 hours at a temperature from -50°C to -35°C under a pressure from 5 Pa to 10 Pa; and
drying the mixture for from 2 hours to 12 hours at a temperature from 10°C to 20°C under a pressure from 5 Pa to 10 Pa.

13. The method of claim 12 wherein the meningococcus C (Men C) immunogen is MenC-CRM 197.

14. A method for lyophilization of a meningococcus C (Men C) immunogen comprising;
dissolving a meningococcus C (Men C) immunogen in a dissolution buffer comprising at least one amorphous excipient and an amorphous buffer to form a mixture;
loading the mixture at 10°C under 1000 Pa of pressure;
freezing the mixture for 2 hours to a temperature of -50°C under 1000 Pa of pressure;
drying the mixture for 24 hours at a temperature of -35°C under a pressure of 5 Pa; and
drying the mixture for about 8 hours at a temperature of 20°C under a pressure of about 5 Pa.

15. The method of claim 14 wherein the immunogen is Men C-CRM 197.

16. A method for stabilizing a meningococcus C (Men C) vaccine upon lyophilization comprising;
(a) dissolving the meningococcus C (Men C) vaccine in a dissolution buffer comprising at least one amorphous excipient and an amorphous buffer to form a mixture, and
(b) lyophilizing the mixture.

17. The method of claim 16 wherein the meningococcus C (Men C) vaccine is MenC-CRM 197.

18. A meningococcus C (Men C) vaccine stabilized according to the method of claim 16 comprising at least one amorphous excipient and at least one amorphous buffer.

## Patentansprüche

1. Auflösungspuffer, umfassend mindestens einen amorphen Excipienten, mindestens einen amorphen Puffer und mindestens ein Meningococcus C (MenC)-Immunogen.

2. Auflösungspuffer nach Anspruch 1, wobei der amorphe Puffer ein organischer Puffer ist.

3. Auflösungspuffer nach Anspruch 2, wobei der organische Puffer ausgewählt ist aus der Gruppe bestehend aus Imidazolpuffer und Histidinpuffer.

4. Auflösungspuffer nach Anspruch 1, wobei der amorphe Excipient ein Zucker ist.

5. Auflösungspuffer nach Anspruch 4, wobei der Zucker Saccharose ist.

6. Auflösungspuffer nach einem der vorhergehenden Ansprüche, wobei das Meningococcus C (MenC)-Immunogen MenC-CRM 197 ist.

7. Verfahren zum Stabilisieren eines oder mehrerer Meningococcus C (MenC)-Immunogene beim Lyophilisieren, umfassend:
(a) Auflösen des Meningococcus C (MenC)-Immunogens in einem Auflösungspuffer, der mindestens einen amorphen Excipienten und einen amorphen Puffer umfasst, um ein Gemisch zu erzeugen, und
(b) Lyophilisieren des Gemischs.

8. Verfahren nach Anspruch 7, wobei das Meningococcus C (MenC)-Immunogen MenC-CRM 197 ist.

9. Lyophilisierte Zusammensetzung, die gemäß dem Verfahren nach Anspruch 7 stabilisiert ist und die mindestens einen amorphen Excipienten, mindestens einen amorphen Puffer und mindestens ein Meningococcus C (MenC)-Immunogen umfasst.

10. Verfahren zum Lagern eines oder mehrerer Meningococcus C (MenC)-Immunogene, umfassend:
(a) Auflösen des Meningococcus C (MenC)-Immunogens in einem Auflösungspuffer, der mindestens einen amorphen Excipienten und einen amorphen Puffer umfasst, um ein Gemisch zu erzeugen, und
(b) Lyophilisieren des Gemischs.

11. Verfahren nach Anspruch 10, wobei das Meningococcus C (MenC)-Immunogen MenC-CRM 197 ist.

12. Verfahren zum Lyophilisieren eines Meningococcus C (MenC)-Immunogens, umfassend:
Auflösen eines Meningococcus C (MenC)-Immunogens in einem Auflösungspuffer, der mindestens einen amorphen Excipienten und einen amorphen Puffer umfasst, um ein Gemisch zu erzeugen;
Laden des Gemischs bei 10°C bis 20°C unter einem Druck von 1000 Pa bis 2000 Pa;
Einfrieren des Gemischs für 1 bis 4 Stunden auf eine Temperatur von -70°C bis -50°C unter einem Druck von 1000 Pa bis 2000 Pa;
Trocknen des Gemischs für 18 bis 30 Stunden bei einer Temperatur von -50°C bis -35°C unter einem Druck vom 5 Pa bis 10 Pa; und
Trocknen des Gemischs für 2 bis 12 Stunden bei einer Temperatur von 10°C bis 20°C unter einem Druck von 5 bis 10 Pa.

13. Verfahren nach Anspruch 12, wobei das Meningococcus C (MenC)-Immunogen MenC-CRM 197 ist.

14. Verfahren zum Lyophilisieren eines Meningococcus C (MenC)-Immunogens, umfassend:
Auflösen eines Meningococcus C (MenC)-Immunogens in einem Auflösungspuffer, der mindestens einen amorphen Excipienten und einen amorphen Puffer umfasst, um ein Gemisch zu erzeugen;
Laden des Gemischs bei 10°C unter einem Druck von 1000 Pa;
Einfrieren des Gemischs für 2 Stunden auf eine Temperatur von -50°C unter einem Druck von 1000 Pa;
Trocknen des Gemischs für 24 Stunden bei einer Temperatur von -35°C unter einem Druck von 5 Pa; und
Trocknen des Gemischs für etwa 8 Stunden bei einer Temperatur von 20°C unter einem Druck von etwa 5 Pa.

15. Verfahren nach Anspruch 14, wobei das Immunogen MenC-CRM 197 ist.

16. Verfahren zum Stabilisieren eines Meningococcus C (MenC)-Impfstoffs beim Lyophilisieren, umfassend:
(a) Auflösen des Meningococcus C (MenC)-Impfstoffs in einem Auflösungspuffer, der mindestens einen amorphen Excipienten und einen amorphen Puffer umfasst, um ein Gemisch zu erzeugen, und
(b) Lyophilisieren des Gemischs.

17. Verfahren nach Anspruch 16, wobei der Meningococcus C (MenC)-Impfstoff MenC-CRM 197 ist.

18. Meningococcus C (MenC)-Impfstoff, stabilisiert gemäß dem Verfahren nach Anspruch 16, der mindestens einen amorphen Excipienten und mindestens einen amorphen Puffer umfasst.

## Revendications

1. Tampon de dissolution comprenant au moins un excipient amorphe, au moins un tampon amorphe, et au moins un immunogène du méningocoque C (MenC).

2. Tampon de dissolution selon la revendication 1, dans lequel le tampon amorphe est un tampon organique.

3. Tampon de dissolution selon la revendication 2, dans lequel le tampon organique est choisi dans le groupe constitué par un tampon d'imidazole et un tampon d'histidine.

4. Tampon de dissolution selon la revendication 1, dans lequel l'excipient amorphe est un sucre.

5. Tampon de dissolution selon la revendication 4, dans lequel le sucre est le saccharose.

6. Tampon de dissolution selon l'une quelconque des revendications précédentes, dans lequel l'immunogène du méningocoque C (MenC) est MenC-CRM 197.

7. Procédé de stabilisation d'un ou de plusieurs immunogènes du méningocoque C (MenC) lors de la lyophilisation comprenant :
(a) dissoudre l'immunogène du méningocoque C (MenC) dans un tampon de dissolution comprenant au moins un excipient amorphe et un tampon amorphe pour former un mélange, et
(b) lyophiliser le mélange.

8. Procédé selon la revendication 7, dans lequel l'immunogène du méningocoque C (MenC) est MenC-CRM 197.

9. Composition lyophilisée stabilisée selon le procédé de la revendication 7, comprenant au moins un excipient amorphe, au moins un tampon amorphe, et au moins un immunogène du méningocoque C (MenC).

10. Procédé de stockage d'un ou de plusieurs immunogènes du méningocoque C (MenC) comprenant :
(a) dissoudre l'immunogène du méningocoque C (MenC) dans un tampon de dissolution comprenant au moins un excipient amorphe et un tampon amorphe pour former un mélange, et
(b) lyophiliser le mélange.

11. Procédé selon la revendication 10, dans lequel l'immunogène du méningocoque C (MenC) est MenC-CRM 197.

12. Procédé de lyophilisation d'un immunogène du méningocoque C (MenC) comprenant :
■ dissoudre un immunogène du méningocoque C (MenC) dans un tampon de dissolution comprenant au moins un excipient amorphe et un tampon amorphe pour former un mélange ;
■ charger le mélange de 10°C à 20°C sous une pression de 1000 Pa à 2000 Pa ;
■ congeler le mélange pendant 1 heure à 4 heures à une température de -70°C à -50°C sous une pression de 1000 Pa à 2000 Pa ;
■ sécher le mélange pendant 18 heures à 30 heures à une température de -50°C à -35°C sous une pression de 5 Pa à 10 Pa ; et
■ sécher le mélange pendant 2 heures à 12 heures à une température de 10°C à 20°C sous une pression de 5 Pa à 10 Pa.

13. Procédé selon la revendication 12, dans lequel l'immunogène du méningocoque C (MenC) est MenC-CRM 197.

14. Procédé de lyophilisation d'un immunogène, du méningocoque C (MenC) comprenant :
■ dissoudre un immunogène du méningocoque C (MenC) dans un tampon de dissolution comprenant au moins un excipient amorphe et un tampon amorphe pour former un mélange ;
■ charger le mélange à 10°C sous une pression de 1000 Pa ;
■ congeler le mélange pendant 2 heures à une température de -50°C sous une pression de 1000 Pa ;
■ sécher le mélange pendant 24 heures à une température de -35°C sous une pression de 5 Pa ; et
■ sécher le mélange pendant environ 8 heures à une température de 20°C sous une pression d'environ 5 Pa.

15. Procédé selon la revendication 14, dans lequel l'immunogène du méningocoque C (MenC) est MenC-CRM 197.

16. Procédé de stabilisation d'un vaccin contre le méningocoque C (MenC) lors de la lyophilisation comprenant :
(a) dissoudre le vaccin contre le méningocoque C (MenC) dans un tampon de dissolution comprenant au moins un excipient amorphe et un tampon amorphe pour former un mélange, et
(b) lyophiliser le mélange.

17. Procédé selon la revendication 16, dans lequel le vaccin contre le méningocoque C (MenC) est MenC-CRM 197.

18. Vaccin contre le méningocoque C (MenC) stabilisé selon le procédé de la revendication 16 comprenant au moins un excipient amorphe et au moins un tampon amorphe.
